**Europäisches Patentamt**

(19) **European Patent Office**

**Office européen des brevets**

(11) Veröffentlichungsnummer: **0 264 554 B1**

(12) **EUROPÄISCHE PATENTSCHRIFT**

(45) Veröffentlichungstag der Patentschrift: 24.10.90

(51) Int. Cl.⁵: **A61M 16/00**, A61M 16/01, G01N 37/00

(21) Anmeldenummer: **87111373.4**

(22) Anmeldetag: **06.08.87**

(54) **Vorrichtung zur automatischen Kalibrierung eines Gassensors.**

(30) Priorität: 26.09.86 DE 3632698

(43) Veröffentlichungstag der Anmeldung: 27.04.88 Patentblatt 88/17

(45) Bekanntmachung des Hinweises auf die Patenterteilung: 24.10.90 Patentblatt 90/43

(84) Benannte Vertragsstaaten: DE FR GB SE

(56) Entgegenhaltungen:
US-A- 3 166 676
US-A- 3 281 595
US-A- 3 465 753

(73) Patentinhaber: **Drägerwerk Aktiengesellschaft, Moislinger Allee 53-55, D-2400 Lübeck 1(DE)**

(72) Erfinder: **Bayerlein,Jörg,Dr.Dipl.-Ing., Waldenburger Strasse 6, D-2406 Stockelsdorf(DE)**

ACTORUM AG

**Beschreibung**

Die Erfindung betrifft eine Vorrichtung mit einer Trägergasleitung, einer Versorgungsleitung, einer Dosiereinheit, einem Sensor und Umschaltern, zur Bestimmung des Anteils einer von der Dosiereinheit in die Trägergasleitung eingebrachten Gaskomponente durch den der Dosiereinheit nachgeschalteten, in der Versorgungsleitung angeordneten Sensor, welchem zu seiner Kalibrierung durch die Umschalter ein komponentenfreies Gas zuführbar ist.

Eine derartige Vorrichtung ist aus der US-A 34 65 753 bekanntgeworden.

Bei der bekannten Vorrichtung wird ein mit einer Gaskomponente, hier Narkosegas, angereichertes Trägergas in eine Versorgungsleitung eingespeist, wobei der Anteil der in das Trägergas abgegebenen Gaskomponente durch einen nachgeschalteten Sensor überwacht wird. Zu Beginn einer Messung und in periodischen Abständen während der Messung muß der Sensor kalibriert werden. Zu diesem Zwecke sind in die Versorgungsleitung jeweils vor und hinter dem Sensor Umschaltventile angeordnet, die jeweils an einen Einlaß und Auslaß für das der Kalibrierung dienende Kalibriergas angeschlossen sind. Während des Kalibriervorganges werden die Umschaltventile derart betätigt, daß der Sensor von der Versorgungsleitung abgetrennt und stattdessen mit einem Kalibriergas gespült wird, welches die nachzuweisende Gaskomponente nicht enthalten darf. Das Kalibriergas kann zum Beispiel Luft sein.

Bei der bekannten Vorrichtung erweist es sich als Nachteil, daß zum Zwecke der Zuführung von Kalibriergas separate Gasanschlüsse vorgesehen sein müssen, welche lediglich während des Kalibriervorganges benötigt werden. Zusätzlich muß für eine entsprechende Bevorratung an Kalibriergas gesorgt sein. Weiterhin wird die Brauchbarkeit der bekannten Vorrichtung dadurch beeinträchtigt, daß während der Dauer des Kalibriervorganges der Trägergasstrom zu der Dosiereinheit unterbrochen ist, so daß ein Hinzudosieren von beispielsweise Narkosegas während des Kalibriervorganges nicht erfolgen kann. Dies kann beispielsweise während einer Narkose zur Folge haben, daß die Narkosegaskonzentrationen während des Kalibriervorganges unerwünscht absinken kann.

Ähnliche Überlegungen sind auch für den Fall anzustellen, wenn zum Beispiel durch Hinzudosieren einer oder mehrerer Gaskomponenten in ein Trägergas ein Gasgemisch in der Versorgungsleitung erzeugt werden soll, dessen Zusammensetzung überwacht werden und auch während des Kalibriervorganges des Sensors unverändert bleiben muß.

Der vorliegenden Erfindung liegt somit die Aufgabe zugrunde, eine Vorrichtung der genannten Art so zu verbessern, daß eine Kalibrierung des Sensors ohne Beeinflussung der Dosierung und ohne Bereithaltung und anschließende Beseitigung eines zusätzlichen Kalibriergases erfolgen kann.

Zur Lösung der Aufgabe wird der Sensor zur Kalibrierung durch die Umschalter an die Trägergasleitung, und die Dosiereinheit strömungsabwärts dem Sensor nachgeschaltet an die Versorgungsleitung angeschlossen.

Die Vorteile der Erfindung sind hauptsächlich darin zu sehen, daß die bei der Messung vorliegende Reihenfolge der Durchströmung von Dosiereinheit und Sensor zum Zwecke der Kalibrierung nur umgekehrt zu werden braucht. Die Dosiereinheit erhält dann nach wie vor die notwendige Menge an Trägergas, dem sie die geforderte Gaskomponente in entsprechender Menge hinzudosieren kann. Während dieser Zeit wird der Sensor mit dem frischen Trägergas, welches die zu messende Gaskomponente noch nicht enthält, durchströmt, so daß eine Kalibrierung möglich ist. Währenddessen bleibt die Konzentration der laufend in das Trägergas dosierten Gaskomponente in vorbestimmter Höhe erhalten. Ferner kann auf eine zusätzliche Kalibriergasquelle verzichtet werden, und es brauchen entsprechende Kalibriergasanschlüsse für Zufuhr und Ableitung nicht vorgesehen und beschaltet zu werden. Nach Abschluß der Kalibration wechseln die Umschalter in ihre ursprüngliche Schaltstellung zurück, wobei die übliche zur Messung notwendige Reihenfolge von Dosiereinheit und Sensor wiederhergestellt ist.

Die Umkehrung der Durchströmungsreihenfolge geschieht zweckmäßigerweise dadurch, daß die Dosiereinheit und der Sensor miteinander verbunden und die Trägergasleitung über einen Zuflußumschalter zur Messung mit dem Eingang der Dosiereinheit oder zur Kalibrierung mit einer an den Ausgang des Sensors angeschlossenen Sensorumwegleitung, sowie die Versorgungsleitung über einen Abflußumschalter zur Messung mit dem Ausgang des Sensors oder zur Kalibrierung mit einer an den Eingang der Dosiereinheit angeschlossenen Dosierumwegleitung verbindbar sind. Diese Schaltungsanordnung erlaubt eine Verwirklichung der Erfindung mit einfachsten Mitteln, weil Dosiereinheit und Sensor als Gruppe umgeschaltet werden. Dabei wird außer der Reihenfolge der Durchströmung auch die Strömungsrichtung in den einzelnen Elementen (Dosiereinheit, Sensor) umgekehrt. Diese Schaltungsanordnung ist deshalb dann vorteilhaft anwendbar, wenn die Funktionen von Dosiereinheit und Sensor unabhängig davon sind, in welcher Richtung das Trägergas sie durchströmt. Sie bietet ferner den Vorteil, daß auch bei Verwendung voneinander unabhängiger Umschalter selbst im Fehlerfalle, bei dem ein Umschalter die Meßstellung und der andere die Kalibrierstellung einnimmt, keine Unterbrechung des Trägergasflusses eintritt.

Für den Fall, daß die Funktion von Dosiereinheit und Sensor nur dann gewährleistet ist, wenn sie immer nur in einer Richtung durchströmt werden, ist es zweckmäßig, daß der Eingang der Dosiereinheit über einen Dosierzufluß-Umschalter zur Messung mit der Trägergasleitung oder zur Kalibrierung mit dem Ausgang des Sensors , daß der Eingang des Sensors über einen Sensorzufluß-Umschalter zur Messung mit der Trägergasleitung oder zur Kalibrierung mit dem Ausgang der Dosiereinheit, sowie daß die Versorgungsleitung über einen Abflußumschalter zur Messung mit dem Ausgang des Sensors oder zur Kalibrierung mit dem Ausgang der Dosiereinheit verbindbar sind. Mit nur drei Umschaltern wird dabei erreicht, daß bei der Umkehrung der Durchströmungsreihenfolge die Strömungsrichtung in den einzelnen Elementen erhalten bleibt.

Die Umschalter können für den Fall, daß die Funktion von Dosiereinheit und Sensor unabhängig von ihrer Druchströmungsrichtung ist, vorteilhafterweise aus einem 4/2-Wegeventil bestehen. In der Meßstellung des Wegeventils werden die hintereinander geschalteten Dosiereinheit und Sensor direkt mit der Trägergasleitung und der Versorgungsleitung verbunden. In der Kalibrierstellung des Wegeventils sind die Verbindungswege dann im Wegeventil gekreuzt. Das Wegeventil wirkt dadurch als Anschlußtauscher, d. h. es vertauscht in seinen Schaltstellungen die Anschlüsse der umzuschaltenden Gruppe, Eingang der Dosiereinheit und Ausgang des Sensors, in ihrer Zuordnung zu den Leitungen Trägergasleitung und Versorgungsleitung. Mit der Umkehrung der Druchströmungsreihenfolge ist eine Kalibrierung des Sensors mit Trägergas möglich.

Für den Fall, daß die Funktionsweise von Dosiereinheit und Sensor abhängig von der Durchströmungsrichtung des Trägergases ist, stehen dem Fachmann mehrere Lösungen zu Gebote, die sich nach Art und Anzahl der eingesetzten Ventile unterscheiden. Eine besonders einfache Anordnung, die das Ziel mit geringem Aufwand erreicht, ergibt sich dadurch, daß die erforderlichen Umschalter zu zwei, an eine gemeinsame Steuerung angeschlossenen Wegeventilen mit in der einen Schaltstellung gekreuzten Wegen zusammengefaßt sind, die einerseits mit der Trägergasleitung und einer Verbindungsleitung zwischen den beiden Wegeventilen bzw. mit der Versorgungsleitung und der Verbindungsleitung, und andererseits mit jeweils einem der Anschlüsse von Dosiereinheit und Sensor verbunden sind.

Wenn auch bei der vorstehenden Erläuterung von gasförmigen Komponenten ausgegangen wurde, so läßt sich die Vorrichtung in analoger Weise für die Messung und Kalibrierung flüssiger Komponenten, welche einem Flüssigkeitsstrom zudosiert werden, anwenden.

Ausführungsbeispiele der Erfindung werden anhand der schematischen Zeichnung dargestellt und im folgenden näher erläutert. Es zeigen als Blockschaltbilder jeweils in der Kalibrierstellung:

Fig. 1 eine Vorrichtung mit wechselnder Strömungsrichtung der Elemente,

Fig. 2 eine weitere Ausführung der Vorrichtung mit gleichbleibender Strömungsrichtung in den Elementen,

Fig. 3 eine weitere Ausführungsform der Vorrichtung nach Fig. 1 mit Wegeventil,

Fig. 4 eine weitere Ausführungsform der Vorrichtung nach Fig. 2 mit Wegeventilen.

In den dargestellten Figuren sind eine Dosiereinheit (5) und ein Sensor (6) in Reihe geschaltet. Dabei ist der Eingang (51) der Dosiereinheit (5) mit einer Trägergasleitung (1) über den Zuflußumschalter (7) verbindbar. Der Ausgang (61) des Sensors (6) ist über einen Abflußumschalter (9) mit der Versorgungsleitung (3) verbunden. Zur Bestimmung des Anteils einer von der Dosiereinheit in die Dosiergasleitung eingebrachten Gaskomponente sind die Umschalter (7, 9) in die gestrichelt dargestellte Meßstellung geschaltet. Dabei ist dann die Trägergasleitung (1) mit dem Eingangsanschluß (11) und der Ausgangsanschluß (12) mit der Versorgungsleitung (3) verbunden. Die entsprechenden Kalibrieranschlüsse (8, 10) der Umschalter (7, 9) sind geöffnet. Das Trägergas aus der Trägergasleitung (1) durchströmt die Dosiereinheit (5) und wird dort mit einer von der Dosiereinheit (5) einzubringenden Gaskomponente angereichert, welche durch den nachgeschalteten Sensor (6) nachweisbar ist. Das von der Dosiereinheit (5) mit einer Gaskomponente angereicherte Trägergas wird einer Versorgungsleitung (3) zugeführt, von wo aus es einem nicht dargestellten Verbraucher zugeleitet werden kann.

Die Dosiereinheit (5) kann beispielsweise ein Verdunster sein, der von dem Trägergas durchströmt wird, oder sie kann von einer Kolbendosierpumpe gebildet sein, welche vorgebbare Mengen einer Gaskomponente in das Trägergas einbringt. Eine Kolbendosierpumpe könnte beispielsweise auch Flüssigkeit in das Trägergas eindosieren, welche im Strömungsverlauf der Dosiereinheit verdunstet. Als Sensor (6) können Nachweiselemente bekannter Bauart, welche auf unterschiedlichen Meßprinzipien beruhen, eingesetzt werden. So sind zum Beispiel Infrarotgasdetektoren einsetzbar oder auch Sensoren zur Wärmeleitfähigkeitsmessung eines Gases. Der Medientransport kann entweder über einen geeigneten Druck in der Trägergasleitung (1) hervorgerufen werden, oder es ist ein nicht dargestelltes Förderelement, zum Beispiel eine Förderpumpe, in die Trägergasleitung (1) oder auch in die Versorgungsleitung (3) einsetzbar.

Die Trägergasleitung (1) ist am Kalibrieranschluß (8) über eine Sensorumwegleitung (2) mit dem Ausgangsanschluß (12) des Sensors (6) verbindbar. Dabei ist der Zuflußumschalter (7) in der ausgezogenen Linienführung in Kalibrierstellung gebracht. Der Abflußumschalter (9) ist ebenfalls, dargestellt durch den ausgezogenen Pfeil, in Kalibrierstellung gebracht. Der Eingangsanschluß (11) ist dann über eine Dosierumwegleitung (4), den Kalibrieranschluß (10) und den Abflußumschalter (9) mit der Versorgungsleitung (3) verbunden. In der dargestellten Kalibrierstellung wird das Trägergas über die Trägergasleitung (1) und die Sensorumwegleitung (2) direkt an den Sensorausgang (61) geleitet, durchströmt den Sensor (6) und die Verbindungsleitung (56) zwischen Sensor (6) und Dosiereinheit (5), wonach die Einspeisung der zu dosierenden Gaskomponente in das Trägergas durch die Dosiereinheit (5) erfolgt. Danach verläßt das mit der eindosierten Gaskomponente versehene Trägergas die Dosiereinheit (5) über den Dosiereingang (51) und die Dosierumwegleitung (4) und wird über den Abflußumschalter (9) in die Versorgungsleitung (3) eingespeist.

Die Reihenfolge der Durchströmung von Dosiereinheit (5) und Sensor (6) wird also in der Kalibrierstellung gegenüber der Meßstellung des Gerätes, unter gleichzeitiger Umkehr der Strömungsrichtung, umgekehrt, so daß der Sensor (6) in der Kalibrierstellung von reinem Trägergas durchströmt wird, welches frei von der durch die Dosiereinheit (5) einzubringenden Gaskomponente ist. Es kann somit eine Null-Kalibrierung mit Hilfe einer nicht dargestellten Sensorkalibriereinheit durchgeführt werden, ohne daß die Dosierung einer gewünschten Gaskomponente in das Trägergas unterbrochen werden muß. Nach abgeschlossener Kalibrierung werden die Umschalter (7, 9) in ihre durch die gestrichelt gezeichneten Pfeile dargestellte Meßstellung zurückgeführt.

Eine solche Umschaltung zwischen Meßstellung und Kalibrierstellung über jeweils einen Umschalter am Dosiereingang (51) und am Sensorausgang (61) ist dann vorteilhafterweise einsetzbar, wenn die Funktion von Dosiereinheit (5) und Sensor (6) unabhängig ist von ihrer jeweiligen Durchströmungsrichtung. Ist die Funktionsfähigkeit von Dosiereinheit (5) und Sensor (6) nur in einer Strömungsrichtung gewährleistet, muß diese sowohl während der Meßstellung als auch während der Kalibrierstellung der jeweiligen Umschalter gesichert sein.

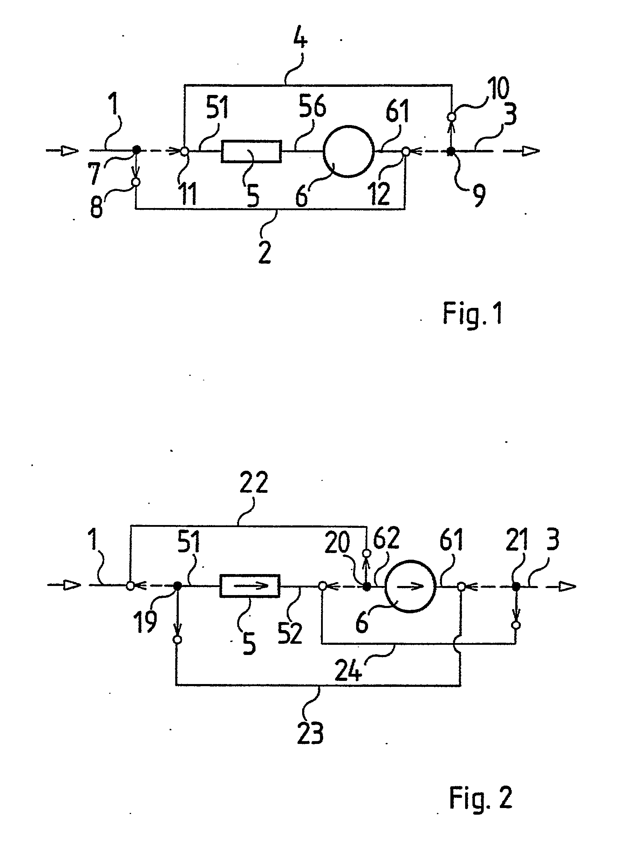

Entsprechend dem Beispiel nach Fig. 2 sind dazu drei Umschalter (19, 20, 21) notwendig. In der mit den gestrichelten Pfeilen der Umschalter (19, 20, 21) dargestellten Meßstellung durchströmt das Trägergas, genauso wie im Beispiel nach Fig. 1, zunächst die Dosiereinheit (5) und anschließend den Sensor (6) in der in ihnen durch Pfeile angedeuteten Richtung und wird der Versorgungsleitung (3) zugeführt. In der durch die ausgezogenen Pfeile der Umschalter (19, 20, 21) dargestellten Kalibrierstellung wird die Trägergasleitung (1) über eine Sensorumwegleitung (22) und den Sensorzufluß-Umschalter (20) mit dem Eingang (62) des Sensors (6) verbunden. Der Ausgang (61) des Sensors (6) ist über die Dosierumwegleitung (23) und den Dosierzufluß-Umschalter (19) mit dem Dosiereingang (51) verbunden. Der Ausgang (52) der Dosiereinheit (5) ist über eine weitere Dosierumwegleitung (24) und Abflußumschalter (21) an die Versorgungsleitung (3) angeschlossen.

Mit dieser Anordnung ist die Durchströmungsrichtung (innere Pfeile) der Dosiereinheit (5) sowie des Sensors (6) in Kalibrierstellung der Umschalter (19, 20, 21) dieselbe, wie sie während der Meßstellung der Umschalter (19, 20, 21) (gestrichelt dargestellte Pfeile) vorliegt.

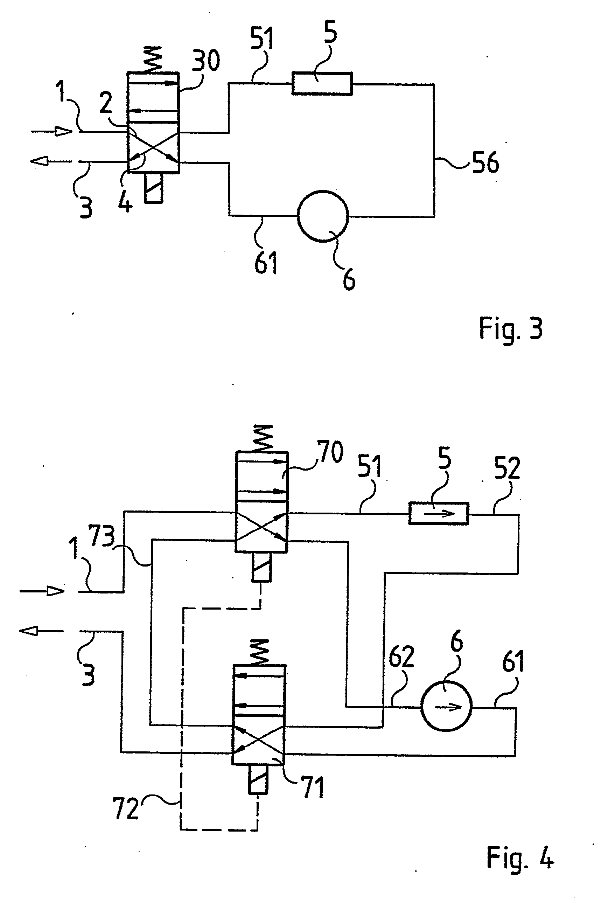

Eine besondere einfache Ausführungsform wird nach Fig. 3 erreicht, wenn die Umschalter (7, 9) durch ein in Kalibrierstellung dargestelltes 4/2-Wegeventil (30) verwirklicht werden. In besonders einfacher Weise sind dabei die Umwegleitungen (2,4) durch die sich in Kalibrierstellung überkreuzenden Wege des 4/2-Wegeventils (30) gebildet. Damit dient es als Anschlußtauscher, indem es zwischen den Schaltstellungen die Zuordnung der miteinander verbundenen Leitungen (1, 3, 51, 61) vertauscht.

Die übrigen Ziffern entsprechen denen aus Fig. 1. Auch diese Ausführungsform ist geeignet für Dosiereinheit (5) und Sensor (6), welche in beiden Richtungen durchströmt werden können.

Für den Fall, daß Dosiereinheit (5) und Sensor (6) zur Erfüllung ihrer Funktion nur in einer Richtung durchströmt werden dürfen, kann die Schaltung nach Fig. 4 mit zwei gleichartigen 4/2-Wegeventilen (70,71) aufgebaut sein, bei denen die Wege in der Kalibrierstellung gekreuzt sind. Auf der einen Seite des Eingangs-Wegeventils (70) sind die Trägergasleitung (1) und eine Verbindungsleitung (73) zum Ausgangs-Wegeventil (71), auf seiner anderen Seite der Dosiereingang (51) der Dosiereinheit (5) sowie der Eingang (62) des Sensors (6) angeschlossen; und auf der einen Seite des Ausgangs-Wegeventils (71) sind die Versorgungsleitung (3) und die Verbindungsleitung (73), auf seiner anderen Seite der Ausgang (52) der Dosiereinheit (5) sowie der Ausgang (61) des Sensors (6) angeschlossen. Die Strömung verläuft dann in der dargestellten Kalibrierstellung von der Trägergasleitung (1) über das Eingangs-Wegeventil (70), den Sensor (6) vom Eingang zum Ausgang, die Wegeventile (71, 70), die Dosiereinheit (5) vom Eingang zum Ausgang und das Ausgangs-Wegeventil (71) zur Versorgungsleitung (3). Nach erfolgter Kalibrierung des Sensors (6) werden die Wegeventile (70, 71) über eine gemeinsame Steuerleitung (72), welche mit einer nicht dargestellten Steuereinheit verbindbar ist, in die Meßstellung zurückgeschaltet. In der Meßstellung verläuft die Strömung von der Trägergasleitung (1) über das Eingangs-Wegeventil (70), die Dosiereinheit (5) vom Eingang zum Ausgang, die Wegeventile (71, 70), den Sensor (6) vom Eingang zum Ausgang und das Ausgangs-Wegeventil (71) zur Versorgungsleitung (3).

**Patentansprüche**

1. Vorrichtung zur Bestimmung des Anteils einer von der Dosiereinheit (5) in die Trägergasleitung (1) eingebrachten Gaskomponente durch den der Dosiereinheit (5) nachgeschalteten, in der Versorgungsleitung (3) angeordneten Sensor (6), welchem zu seiner Kalibrierung durch die Umschalter ein komponentenfreies Gas zuführbar ist, dadurch gekennzeichnet, daß zur Kalibrierung der Sensor (6) durch die Umschalter (7, 9, 19, 20, 21, 30, 70, 71) an die Trägergasleitung (1), und die Dosiereinheit (5) strömungsabwärts dem Sensor (6) nachgeschaltet an die Versorgungsleitung (3) angeschlossen ist.

2. Vorrichtung nach Anspruch 1, dadurch gekennzeichnet, daß die Dosiereinheit (5) und der Sensor (6) miteinander verbunden und die Trägergasleitung (1) über einen Zuflußumschalter (7) zur Messung mit dem Eingang (51) der Dosiereinheit (5) oder zur Kalibrierung mit einer an den Ausgang (61) des Sensors (6) angeschlossenen Sensorumwegleitung (2), sowie die Versorgungsleitung (3) über einen Abflußumschalter (9) zur Messung mit dem Ausgang (61) des Sensors (6) oder zur Kalibrierung mit einer an den Eingang (51) der Dosiereinheit (5) angeschlossenen Dosierumwegleitung (4) verbindbar sind.

3. Vorrichtung nach Anspruch 1, dadurch gekennzeichnet, daß der Eingang (51) der Dosiereinheit (5) über einen Dosierzufluß-Umschalter (19) zur Messung mit der Trägergasleitung (1) oder zur Kalibrierung mit dem Ausgang (61) des Sensors (6), daß der Eingang (62) des Sensors (6) über einen Sensorzufluß-Umschalter (20) zur Messung mit dem Ausgang (52) der Dosiereinheit (5) oder zur Kalibrierung mit der Trägergasleitung (1), sowie daß die Versorgungsleitung (3) über einen Abflußumschalter (21) zur Messung mit dem Ausgang (61) des Sensors (6) oder zur Kalibrierung mit dem Ausgang (52) der Dosiereinheit (5) verbindbar sind.

4. Vorrichtung nach Anspruch 2, dadurch gekennzeichnet, daß die beiden Umschalter (7, 9) zu einem Wegeventil (30) als Anschlußtauscher zusammengefaßt und dabei die Sensorumwegleitung und die Dosierumwegleitung durch sich kreuzende Wege (2,4) des Wegeventils (30) gebildet sind.

5. Vorrichtung nach Anspruch 3, dadurch gekennzeichnet, daß die drei Umschalter zu zwei, an eine gemeinsame Steuerung (72) angeschlossenen Wegeventilen (70, 71) mit in der einen Schaltstellung gekreuzten Wegen zusammengefaßt sind, die einerseits mit der Trägergasleitung (1) und einer Verbindungsleitung (73) zwischen den beiden Wegeventilen bzw. mit der Versorgungsleitung (3) und der Verbindungsleitung (73), und andererseits mit jeweils einem der Anschlüsse (51, 52, 61, 62) von Dosiereinheit (5) und Sensor (6) verbunden sind.

## Claims

1. Device with a carrier gas line (1), a supply line (3), a dosing unit (5), a sensor (6) and changeover switches (7, 9; 19, 20, 21; 30; 70, 71) to determine the portion of a gas component, introduced by the dosing unit (5) into the carrier gas line (1), by means of the sensor (6) subsequently wired to the dosing unit (5) and arranged in the supply line (3), to which sensor for its calibration by means of the change-over switches a component-free gas can be supplied, characterized in that for the calibration the sensor (6) is connected by means of the change-over switches (7, 9; 19, 20, 21; 30; 70, 71) to the carrier gas line (1), and the dosing unit (5) subsequently wired in a down-current manner to the sensor (6) is connected to the supply line (3).

2. Device according to claim 1, characterized in that the dosing unit (5) and the sensor (6) are connected to one another, and the carrier gas line (1) can be connected by means of an inflow changeover switch (7) to the input (51) of the dosing unit (5) for measuring or to a sensor by-pass line (2) attached to the output (61) of the sensor (6) for calibration, and the supply line (3) can be connected by means of an outflow change-over switch (9) to the output (61) of the sensor (6) for measuring or to a dosing by-pass line (4) attached to the input (51) of the dosing unit (5) for calibration.

3. Device according to claim 1, characterized in that the input (51) of the dosing unit (5) can be connected by means of a dosing inflow change-over switch (19) to the carrier gas line (1) for measuring or to the output (61) of the sensor (6) for calibration, in that the input (62) of the sensor (6) can be connected by means of a sensor inflow changeover switch (20) to the output (52) of the dosing unit (5) for measuring or to the carrier gas line (1) for calibration, and in that the supply line (3) can be connected by means of an outflow change-over switch (21) to the output (61) of the sensor (6) for measuring or to the output (52) of the dosing unit (5) for calibration.

4. Device according to claim 2, characterized in that the two change-over switches (7, 9) are combined into a directional control valve (30) as connection exchanger and in this respect the sensor by-pass line and the dosing by-pass line are formed by crossing paths (2, 4) of the directional control valve (30).

5. Device according to claim 3, characterized in that the three change-over switches are combined into two directional control valves (70, 71) attached to a common control (72) with paths crossed in the one switch position, which on the one hand are connected to the carrier gas line (1) and a connecting line (73) between the two directional control valves or to the supply line (3) and the connecting line (73), and on the other hand are connected to one of the connections (51, 52, 61, 62) of dosing unit (5) and sensor (6) respectively.

## Revendications

1. Dispositif comportant une conduite de gaz porteur (1), une conduite d'alimentation (3), une unité de dosage (5), un détecteur (6) et des commutateurs (7, 9; 19, 20, 21; 30; 70, 71), destiné à déterminer la proportion d'un composant de gaz, introduit dans la conduite de gaz porteur (1) par l'unité de dosage (5), par le détecteur (6), monté en aval de l'unité de dosage (5), dans la conduite d'alimentation (3), auquel on envoie, pour son calibrage, par les commutateurs, un gaz exempt de composant, caractérisé en ce que pour le calibrage, par les commutateurs (7, 9; 19, 20, 21; 30; 70, 71) le détecteur (6) est raccordé à la conduite de gaz porteur (1) et l'unité de dosage (5), montée en aval du détecteur (6), est raccordée à la conduite d'alimentation (3).

2. Dispositif selon la revendication 1, caractérisé en ce que l'unité de dosage (5) et le détecteur (6) sont reliés entre eux et en ce que la conduite de gaz porteur (1) est reliée, par un commutateur d'alimentation (7), à l'entrée (51) de l'unité de dosage (5), pour la mesure ou à la conduite de contournement du détecteur (2), raccordée à la sortie (61) du détecteur (6), pour le calibrage et en ce que la conduite d'alimentation (3) est reliée, par un commutateur d'évacuation (9), à la sortie (61) du détecteur (6), pour la mesure ou à la conduite de contournement du dosage (4), raccordée à l'entrée (51) de l'unité de dosage (5), pour le calibrage.

3. Dispositif selon la revendication 1, caractérisé en ce que l'entrée (51) de l'unité de dosage (5) est reliée, par un commutateur d'alimentation de dosage (19), à la conduite du gaz porteur (1), pour la mesure ou à la sortie (61) du détecteur (6), pour le calibrage, en ce que l'entrée (62) du détecteur (6) est reliée, par un commutateur d'alimentation de détecteur (20), à la sortie (52) de l'unité de dosage (5), pour la mesure ou à la conduite de gaz porteur (1), pour le calibrage et en ce que la conduite d'alimentation (3) est reliée, par un commutateur d'évacuation (21), à la sortie (61) du détecteur (6), pour la mesure ou à la sortie (52) de l'unité de dosage (5) pour le calibrage.

4. Dispositif selon la revendication 2, caractérisé en ce que les deux commutateurs (7, 9) sont réunis sous la forme d'une vanne de distribution (30), pour servir d'échangeur de raccordement et en ce que la conduite de contournement de détecteur et la conduite de contournement de dosage sont formées par des voies (2, 4), se croisant, de la vanne de distribution (30).

5. Dispositif selon la revendication 3, caractérisé en ce que les trois commutateurs sont réunis dans deux vannes de distribution (70, 71), raccordées à une commande (72) commune, avec des voies se croisant dans une position de commande, lesquelles vannes sont reliées d'une part à la conduite de gaz porteur (1) et à une conduite de liaison (73), entre les deux vannes de distribution ou à la conduite d'alimentation (3) et à la conduite de liaison (73) et sont reliées chacune, d'autre part, à l'un des raccordements (51, 52, 61, 62) de l'unité de dosage (5) et du détecteur (6).

Fig. 1

Fig. 2

Fig. 3

Fig. 4